# EUROPEAN PATENT APPLICATION

(11) **EP 3 597 272 A1**
(43) Date of publication of application: **22.01.2020**
(21) Application number: 19186547.6
(22) Date of filing: 16.07.2019
(51) Int. Cl.: A61Q 5/00, A61Q 5/06, A61Q 5/10, A61K 8/22

(54) **HAIR COSMETIC METHOD EMPLOYING GASESOUS OXYGEN**

(30) Priority: 17.07.2018 IT 201800007261
(71) Applicant: Tricomef S.R.L., 40010 Sala Bolognese (BO) (IT)
(72) Inventor: Montanari, Federico, 40010 Sala Bolognese BO (IT)
(74) Representative: Perani & Partners S.p.A.

(57) **Abstract**

Hair cosmetic method, including the phases of:
a) applying a hair care and beauty treatment to the hair;
b) supplying gaseous oxygen (O₂) to the hair, simultaneously and/or subsequently to the hair care and beauty treatment,
said hair care and beauty treatment being a hair colouring treatment,
wherein the gaseous oxygen has a concentration higher than 80% (v/v) and the gaseous oxygen supply phase lasts between 2 and 20 minutes.

## Description

### FIELD OF THE INVENTION

The present invention relates to a hair cosmetic method involving the use of gaseous oxygen.

### STATE OF THE ART

The oxygen therapy is a technique that uses suitably purified gaseous oxygen for the treatment of face and body skin, as well as for the treatment of hair and scalp.

An index factor for healthy hair is the potential of hair growth from the scalp, a phenomenon in which the amount of oxygen at tissue level is a factor of great importance.

Poor blood circulation in the scalp can in fact cause laziness of hair follicles and slow hair growth.

The purified oxygen supply to the scalp stimulates the local blood circulation and allows a faster renewal of the skin cells; it promotes hair growth in the dermis layer of the scalp as well as the reconstruction of damaged hair from the inside, restoring its recovering ability and refreshing the hair cuticle and cortex.

In fact, oxygen therapy is also currently used to combat baldness and other scalp imbalances.

Among the other applications of concentrated oxygen, there is the treatment of hair aimed at the reconstruction of the correct structure of the hair stem, which can have been damaged by external causes such as cosmetic treatments, atmospheric conditions and pollution.

### SUMMARY OF THE INVENTION

The Applicant has now developed a hair cosmetic method, which involves combining the use of gaseous oxygen (oxygen therapy) with hair care and beauty treatments, such as, for example, treatments carried out by a hairdresser.

The advantage of this combination lies in the fact that the treatment effect is visibly better and lasts longer, even after numerous washes.

The object of the present invention is a hair cosmetic method comprising the steps of:
a) applying a hair care and beauty treatment to the hair;
b) supplying gaseous oxygen (O₂) to the hair, simultaneously and/or subsequently to the hair care and beauty treatment,
said hair care and beauty treatment being a hair colouring treatment,
wherein the gaseous oxygen has a concentration ≥ 80% (v/v) and the gaseous oxygen application phase has a duration comprised between 0.5 and 20 minutes.

### DESCRIPTION OF THE FIGURES

**Figure 1****:** Table 2 shows the values of L*, a* and b* found on the bleached locks used for the test. Tables 3.1-3.3 show the values of L*, a* and b* detected on the control locks, subjected to brown colouring, without being treated with oxygen for 6 minutes. The survey was carried out after 0,5 and 10 washes.
**Figure 2****:** Table 3.4 shows the values of the parameters detected on the control locks, subjected to brown colouring, without being treated with oxygen for 6 minutes. The survey was carried out after 15 washes. Tables 4.1-4.4 report the values of L*, a* and b* detected on the test locks, subjected to brown colouring and treated with oxygen for 6 minutes. The survey was carried out after 0,5, 10 and 15 washes.
**Figure 3****:** Tables 6.1-6.4 show the values of L*, a* and b* detected on the control locks, subjected to brown colouring, without being treated with oxygen for 12 minutes. The survey was carried out after 0 - 15 washes.
**Figure 4****:** Tables 7.1-7.4 show the values of L*, a* and b* detected on the test locks, subjected to brown colouring and treated with oxygen for 12 minutes. The survey was carried out after 0,5, 10 and 15 washes.
**Figure 5****:** Tables 9.1-9.4 show the values of L*, a* and b* detected on the control locks, subjected to red colouring, without being treated with oxygen for 6 minutes. The survey was carried out after 0, 5, 10 and 15 washes.
**Figure 6****:** Tables 10.1-10.4 show the values of L*, a* and b* detected on the test locks, subjected to red colouring and treated with oxygen for 6 minutes. The survey was carried out after 0,5, 10 and 15 washes.
**Figure 7****:** Tables 12.1-12.4 show the values of L*, a* and b* detected on the control locks, subjected to red colouring, without being treated with oxygen for 12 minutes. The survey was carried out after 0,5, 10 and 15 washes.
**Figure 8****:** Tables 13.1-13.4 show the values of L*, a* and b* detected on the test locks, subjected to red colouring and treated with oxygen for 12 minutes. The survey was carried out after 0,5, 10 and 15 washes.

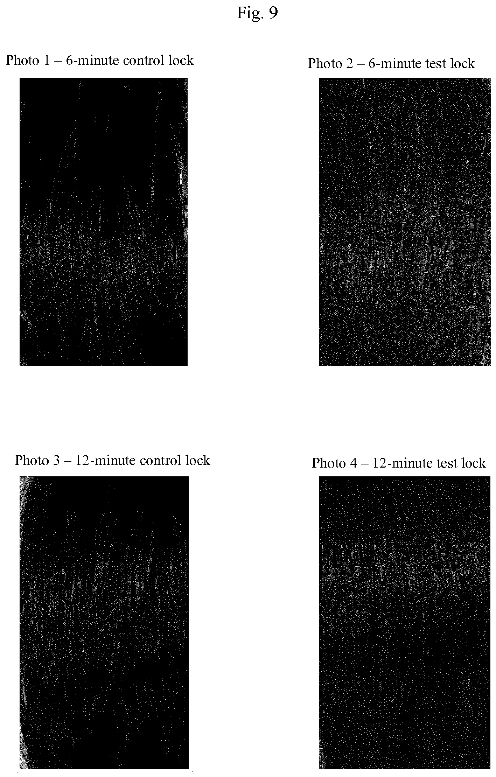

**Figure 9****:** Photo of the brown locks at the end of the colouring test.

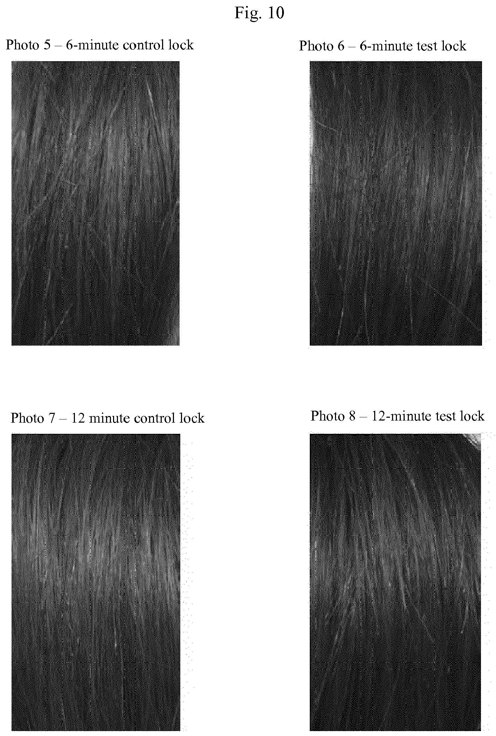

**Figure 10****:** Photo of the red locks at the end of the colouring test.

### DETAILED DESCRIPTION OF THE INVENTION

In the purposes of the present invention, hair care and beauty treatment means any trichological treatment, operable by a hairdresser or by any other expert in the field, which provides the application of a cosmetic composition on the hair or on the scalp, or a manipulation of the hair aimed at imparting a certain shape.

According to a preferred embodiment of the invention, said hair care and beauty treatment is a hair colouring treatment.

According to an alternative embodiment of the invention, which is not the object of the present application, hair care and beauty treatment means a treatment selected in the group consisting of: mask, balm, hair reconstruction, colouring, styling and hairdressing.

Colouring means the technique with which the natural colour of the hair is dyed or bleached. Colouring includes dyes of natural or synthetic origin, such as for example selected among temporary colouring; semi-permanent colouring; permanent colouring and bleaching.

Styling means the technique used to temporarily change the hair shape through the use of the brush, the hair dryer or other hot tools, such as hair straighteners; or permanently through the use of chemical compositions that allow modifying the structure of the hair, acting on the formation of the keratin disulphide bridges.

In order to avoid any excessive damage to the hair, it should be remembered that heat damages the proteins of the cuticle denaturing them. It is therefore very important to pay close attention to the temperature reached by the tools used, which must not be too high.

In the case of the colouring treatment, the method according to the present invention preferably provides that the gaseous oxygen supply to the hair is simultaneous and subsequent to the treatment application.

Instead, in the case of a styling treatment, which is not the object of the present application, the method according to the present invention preferably provides that the gaseous oxygen supply to the hair is simultaneous to the treatment application.

The concentration of gaseous oxygen is preferably between 85% and 99%, more preferably between 93% and 99% (v/v), and even more preferably between 95% and 98% (v/v).

The gaseous oxygen is supplied for a duration preferably comprised between 2 and 20 minutes, more preferably between 6 and 12-minutes.

In particular, in the case of the colouring treatment, the simultaneous supply of gaseous oxygen preferably occurs at the end of the time of pose of the colouring treatment; the supply of subsequent gaseous oxygen preferably occurs after the removal of the colouring treatment.

According to the preferred embodiment, the gaseous oxygen supply is characterized by a flow of between 0.1 and 20 L/min. Preferably, the gaseous oxygen supply is characterized by a flow of between 5 and 9 L/min, more preferably equal to 8 L/min.

Said flow of gaseous oxygen is preferably supplied at a pressure of between 0.1 and 3 bars, more preferably 1 and 3 bars, and still more preferably equal to 2 bars.

### EXAMPLES

Below is an illustrative and non-limiting example of an oxygen therapy treatment according to the present invention.

### 1. Introduction

The aim of the study is the qualitative-quantitative evaluation of a treatment performed on hair locks using the VL-1 device. The comparison is made between control locks and locks subjected to the treatment for 6 minutes or 12 minutes. In particular, the following were evaluated:
- maintenance of the colouring on the locks;
- curl retention (styling).

### 2. Materials

*VL-1 device*: it is made up of a generator that allows sucking the ambient air and, filtering it through two filters composed of a zeolite, transforming it into a gas mixture in which oxygen is present in a concentration equal to 93- 99%. Air is then supplied through a special airbrush on the surface to be treated, in this case on hair locks.

*Hair braids:* the braids used for the colour maintenance test and the gloss evaluation are distinguished from those used for the curl retention evaluation.
- the locks used in the colour maintenance test and the gloss evaluation come from "Italian Virgin Hair" depigmented hair braids, about 16 cm long, purchased by the company Imhair - Palermo. They are composed of smooth, blond, bleached hair. The braids are divided into test locks, weighing about 2.50 g each and held together by elastic cotton.
- the locks used in the retention curl test come from "Italian Virgin Hair" hair braids, about 16 cm long, purchased by the company Stefano Lo Curcio - Manifattura Italiana Capelli - Palermo. They are composed of straight dark brown hair. The braids are divided into test locks, weighing about 2.5-3.5 g, weighed exactly to the second decimal place and held together by elastic cotton.

*Basic shampoo*: all the locks used, both control and treated locks, have been previously washed with a basic shampoo to make the surface equal. The qualitative-quantitative formula is shown in Table 1.

**Table 1**

| **INGREDIENTS** | **INCI** | **%w/w** |
|---|---|---|
| DEMIN. WATER | AQUA | 46.70 |
| ZETESOL LES 3/D | AQUA, SODIUM LAURETH SULPHATE | 48.00 |
| COMPERLAN KD | COCAMIDE DEA | 2.00 |
| FARACIDE HMG | AQUA, SODIUM HYDROXYMETHYLGLICYNATE | 0.80 |
| SODIUM CHLORIDE | SODIUM CHLORIDE | 2.50 |
| | | **100.00** |

For the colour maintenance test, two dyes with different colours were used, both activated by an oxidizing cream in a ratio of 1:1.5.

*Dark brown dye*: VOLUMAGE hair tech colouring cream - dark brown, to be mixed with the oxidizing cream 20 vol. in a ratio of 1:1.5 and to be left for 30' for covering white hair.

*Intense red dye*: VOLUMAGE hair tech colouring cream - intense red, to be mixed with the oxidizing cream 20 vol. in a ratio of 1:1.5 and to be left for 30' for covering white hair.

*Oxidising cream*: VOLUMAGE Oxycream oxidizing cream, 20 volumes.

The following were used for the curl retention test:
Measuring tool: graph paper sheets and certified 1 and 5g E2 class metal weights.

### 3. Methods - Colour and gloss maintenance evaluation

The test involves the colouring of a total of 24 bleached locks by means of two different dyes, dark brown and deep red.

**Colour** - The comparison is made between control locks and locks treated for 6 or 12 minutes with the VL-1 device. The treatment involves the supply of 96% pure oxygen, with a flow of 5 L/min, at a pressure of 1 bar. All the locks were washed using the basic shampoo (Table 1) for about 2 minutes and dried with a common hairdryer (2000 Watts) for 10 minutes.

The following groups, consisting of 3 locks each, are distinguished for both colours:
- Group 1 - 6-minute test locks: the dye was left for the 30 minutes required by the normal procedure and then the locks were treated for 6 minutes with oxygen supplied at maximum flow. Each lock was rinsed with water only, treated again for 6 minutes and finally dried.
- Group 2 - 12-minute test locks: the dye was left for the 30 minutes required by the normal procedure and then the locks were treated for 12 minutes with oxygen supplied at maximum flow. Each lock was rinsed with water only, treated again for 12 minutes and finally dried.
- Group 3 - 6-minute control locks: the dye was left for 36 minutes = 30 minutes required by the normal procedure + 6 minutes to get the same processing time of the treated locks; then rinsing was carried out with water only, followed by drying.
- Group 4 - 12-minute control locks: the dye was left for 42 minutes = 30 minutes required by the normal procedure + 12 minutes to get the same processing time of the treated locks; then rinsing was carried out with water only, followed by drying.

The measurements were carried out by means of a Konica Minolta Chroma Meter CR-400 colourimeter, which performs measurements on a small area (8 mm in diameter). Measurements were taken at 4 different times: time 0, after 5 washes, after 10 washes and after 15 washes. On each lock, three measurements were taken at three different points for a total of 9 measurements per group.

Each wash was carried out for about 2 minutes per group of locks, always using the basic shampoo reported above; after each wash the locks were dried with a common hairdryer.

The tool provides very accurate and reproducible measurements. In this test, every single measurement derives from the average of 3 readings, which is automatically provided by the tool.

The detection system used is called CIELab and the measured parameters correspond to the Cartesian coordinates L*, a*, b*:
- L* = indicates the brightness, expressed as a percentage (0 for black and 100 for white)
- a* = indicates the green-red chromatic component, with values from -60 to +60
- b* = indicates the blue-yellow chromatic component, with values from -60 to +60

***Gloss*** - Comparison is made between control locks and locks treated for 6 minutes with the VL-1 device. The treatment involves the supply of 96% pure oxygen, with a flow of 5 L/min, at a pressure of 1 bar. All the locks were washed using the basic shampoo (Table 1) for about 2 minutes and dried with a common hairdryer (2000 Watts) for 10 minutes.

For both colours, the gloss evaluation was carried out considering only the results obtained by the Group 1 - 6-minute test locks and by the Group 3 - 6-minute control locks.

### 4. Results - Colour Maintenance Evaluation

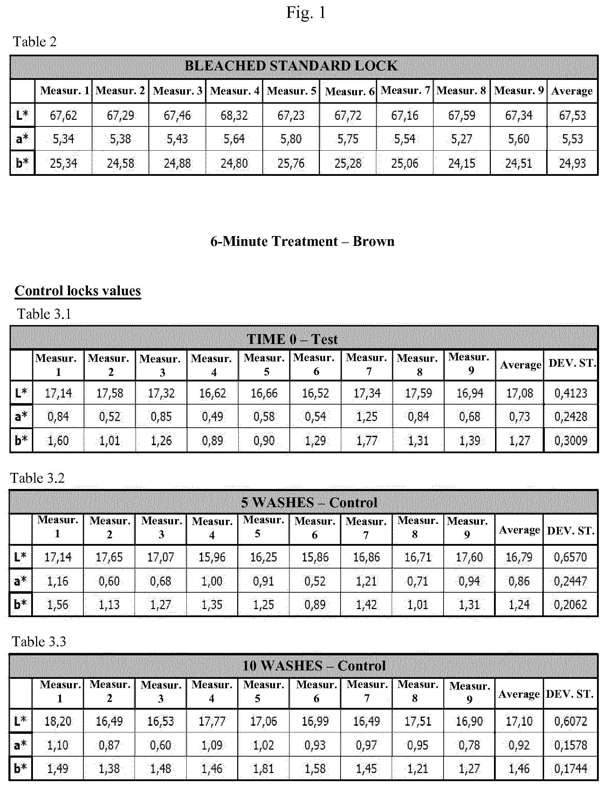

In Table 2, Figure 1 shows the values of L*, a* and b* detected on the bleached locks used for the test.

### 4.1. 6-minute treatment - Brown

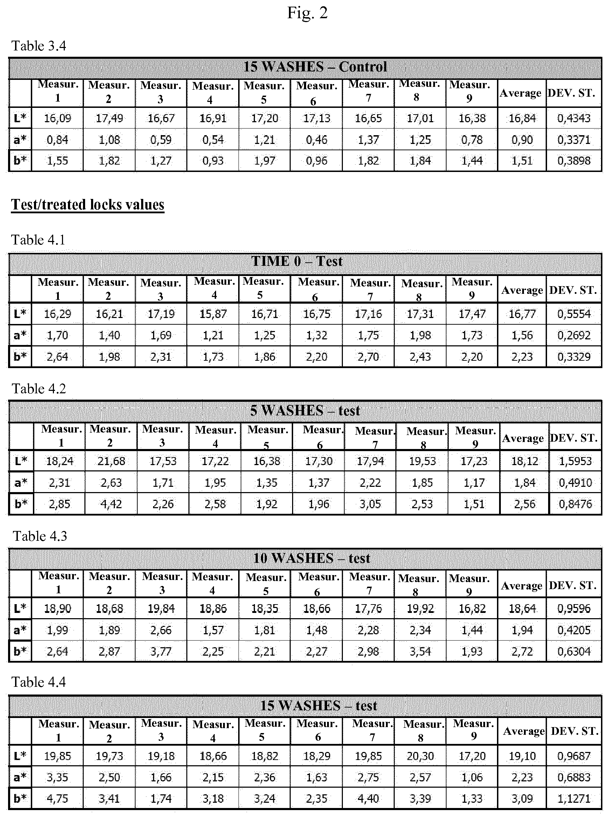

Values of control locks: For the results, see Tables 3.1-3.4. Figure 1.
Values of treated/test locks: For the results, see Tables 4.1-4.4. Figure 2.

***Colour*** - The interpretation of the data relative to the colour relies on the concept of "colour", which is calculated as a ratio a*/b*. The calculated values, based on the averages of a* and b*, for each time are the following:

**Table 5.1**

| **Control locks (6 minutes)** | | | | |
|---|---|---|---|---|
| | **T0** | **5 washes** | **10 washes** | **15 washes** |
| **a*/b*** | 0.577 | 0.691 | 0.633 | 0.597 |

**Table 5.2**

| **Test locks (6 minutes)** | | | | |
|---|---|---|---|---|
| | **T0** | **5 washes** | **10 washes** | **15 washes** |
| **a*/b*** | 0.699 | 0.718 | 0.714 | 0.721 |

In particular, the values obtained at time 0 and after 15 washes are considered for each lock: it is evaluated whether the variation of the colour between treated locks and control locks is significant by applying the t-test function; with p ≤ 0.05 the difference between the values is considered significant.

The comparison between the two groups (treated and control locks) at the initial time and after 15 washes gives this result:
Time 0 washes - test vs control: p = 0.007 -
Time 15 washes - test vs control: p = 0.02 -

The comparison within the same group between the initial time and after 15 washes gives this result:
Control - time 0 washes vs time 15 washes: p = 0.761 -
Test - time 0 washes vs time 15 washes: p = 0.179 -

The variation of the "colour" is therefore significant by comparing the control locks with the treated locks, while it is not significant by comparing the value at time 0 and after 15 washes, both for the control group and for the test group.

This means that in both cases, control and test, there is not a substantial variation in the colour quality of the locks during the washes, but at the same time the treated locks with oxygen have a warmer colour for the entire duration of the test.

It should be noted that the higher the ratio (a*/b*), the more red prevails over yellow in the colour. It is therefore observed that for every time the treated locks have a higher colour value than the control locks. In fact, despite the non-significance of the values, the observed colour is warmer, more intense for the locks subjected to oxygen treatment. The difference may not be statistical due to the non-homogeneity of the locks fibres, which influence the colour reading.

### 4.2. 12-minute treatment - Brown

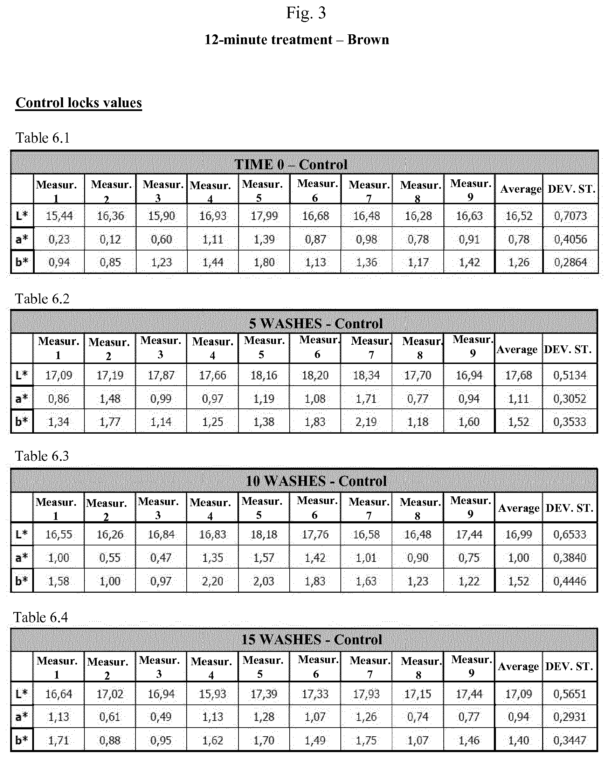

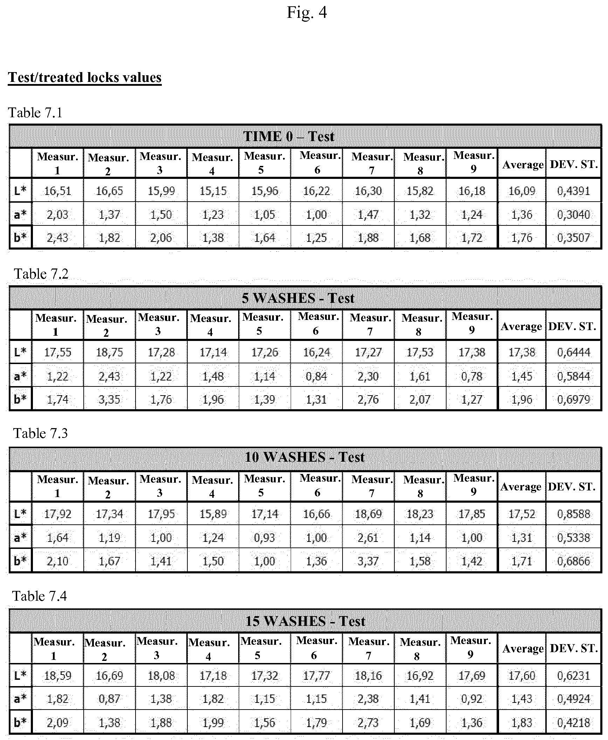

Values of control locks: For the results see Tables 6.1-6.4. Figure 3.
Values of treated/test locks: For the results see tables 7.1-7.4. Figure 4.

Also in this case, the interpretation of the data refers to the value of "colour" (a*/b*).

The values calculated based on the averages of a* and b* for each time are the following:

**Table 8.1**

| **Control locks (12 minutes)** | | | | |
|---|---|---|---|---|
| | **T0** | **5 washes** | **10 washes** | **15 washes** |
| **a*/b*** | 0.616 | 0.730 | 0.659 | 0.671 |

**Table 8.2**

| **Test locks (12 minutes)** | | | | |
|---|---|---|---|---|
| | **T0** | **5 washes** | **10 washes** | **15 washes** |
| **a*/b*** | 0.770 | 0.739 | 0.762 | 0.783 |

The comparison between the two groups (treated and control locks) at the initial time and after 15 washes gives this result:
Time 0 washes - test vs control: p = 0.052 -
Time 15 washes - test vs control: p = 0.033 -

The comparison within the same group between the initial time and after 15 washes gives this result:
Control - time 0 washes vs time 15 washes: p = 0.308 -
Test - time 0 washes vs time 15 washes: p = 0.929 -

As regards the comparison between control locks and treated locks, the variation in colour is virtually not significant at time 0, whereas it is significant after 15 washes; the comparison of the colour values obtained at time 0 and after 15 washes, both for the control locks and for the treated locks, does not show any significant variation. From a visual evaluation, also in this case, it is possible to state that the colour on the treated locks is however warmer and more intense if compared to the untreated locks that show a duller colour.

### 4.3. Comments

In both cases, treatments for 6 and 12 minutes, the values showed no significant differences after 15 washes. However, a greater stability is visible in the "colour" value in the case of treated hair if compared to the control locks, which undergo a greater variation during washing.

Moreover, from a visual evaluation of the colourist expert, it is possible to state that in both cases the colour applied with treatment takes on a warmer and more intense hue than the untreated one, which is duller.

The result emerges both at time 0 and after 15 washes for the 6-minute treatment, whereas it shows only after 15 washes for the 12-minute treatment.

This can also be deduced from the "colour" values, which in all cases are higher than the values of the untreated locks, reflecting a lack of the red component.

### 4.4. 6-minute treatment - Red

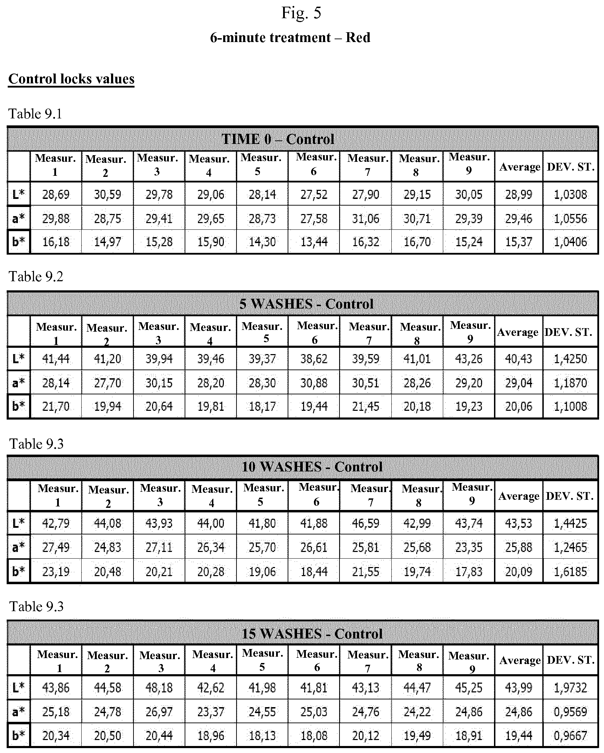

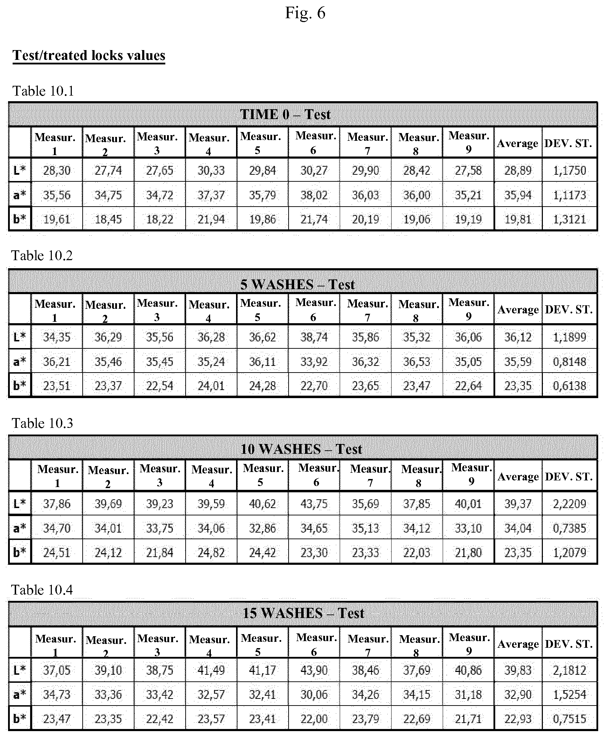

Values of control locks: For the results, see tables 9.1-9.4. Figure 5.
Values of treated/test locks: For the results, see tables 10.1-10.4. Figure 6.

The evaluation of the results obtained mainly relies on the values of a* both for the control locks and for those treated with oxygen. The greater the value of a*, the greater the expression of the red colour.

The average values of a* at different times are as follows:

**Table 11.1**

| **Control locks (6 minutes)** | | | | |
|---|---|---|---|---|
| | **T0** | **5 washes** | **10 washes** | **15 washes** |
| **a*** | 29.46 | 29.04 | 25.88 | 24.86 |

**Table 11.2**

| **Test locks (6 minutes)** | | | | |
|---|---|---|---|---|
| | **T0** | **5 washes** | **10 washes** | **15 washes** |
| **a*** | 35.94 | 35.59 | 34.04 | 32.90 |

In particular, the values obtained at time 0 and after 15 washes are examined for each lock: it is evaluated whether the variation of the parameter a* between treated locks and control locks is significant by applying the t-test function. If the values show a difference, it is considered significant.

The comparison between the two groups (treated and control locks) at the initial time and after 15 washes gives this result:
Time 0 washes - test vs control: p = 3.5 x 10⁻⁶ -
Time 15 washes - test vs control: p = 7 x 10⁻⁷ -

The comparison within the same group between the initial time and after 15 washes gives this result:
Control - time 0 washes vs time 15 washes: p = 1.8 x 10⁻⁵ -
Test - time 0 washes vs time 15 washes: p = 4.1 x 10⁻³ -

As shown by the results, the differences between control locks and treated locks at time 0 and after 15 washes are largely significant. The red is much more intense for the locks subjected to treatment, starting from time 0 until the end of the test.

As regards the variation between time 0 and after 15 washes, it is evident that in both cases, control and test, a significant loss of intensity occurs. This loss is however visually lower for the treated locks with respect to the control ones.

### 4.5. 12-minute treatment - Red

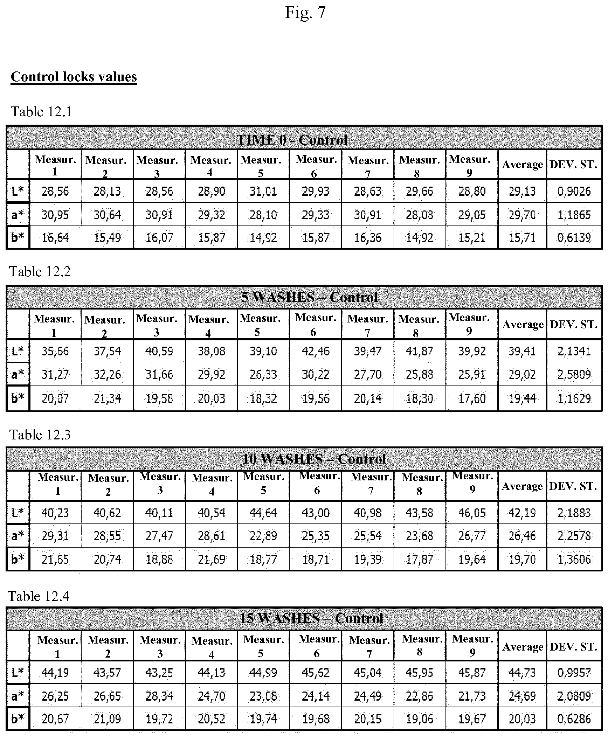

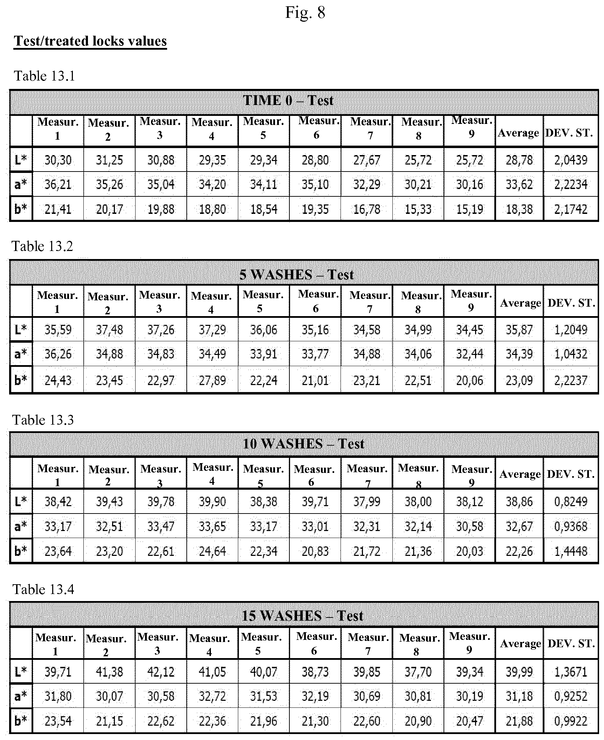

Values of control locks: For the results see Tables 12.1-12.4. Figure 7.
Values of treated/test locks: For the results see Tables 13.1-13.4. Figure 8.

Also in this case reference is made to the parameter a* for the evaluation of the results obtained both for the control locks and for the treated locks. The average values of a* at different times are as follows:

**Table 14.1**

| **Control locks (12 minutes)** | | | | |
|---|---|---|---|---|
| | **T0** | **5 washes** | **10 washes** | **15 washes** |
| **a*** | 29.70 | 29.02 | 26.46 | 24.69 |

**Table 14.2**

| **Test locks (12 minutes)** | | | | |
|---|---|---|---|---|
| | **T0** | **5 washes** | **10 washes** | **15 washes** |
| **a*** | 33.62 | 34.39 | 32.67 | 31.18 |

The comparison between the two groups (treated and control locks) at the initial time and after 15 washes gives this result:
Time 0 washes - test vs control: p = 2.5 x 10⁻⁴ -
Time 15 washes - test vs control: p = 3.1 x 10⁻⁵ -

The comparison within the same group between the initial time and after 15 washes gives this result:
Control - time 0 washes vs time 15 washes: p = 3.9 x 10⁻⁶ -
Test - time 0 washes vs time 15 washes: p = 6.7 x 10⁻³ -

The differences between control locks and treated locks at time 0 and after 15 washes are largely significant. Red is much more intense for the locks subjected to treatment, starting from time 0 until the end of the test.

As regards the variation between time 0 and after 15 washes, in both cases, control and test, a significant loss of intensity occurs. This loss is however visually lower for the treated locks with respect to the control ones.

Hereinafter a comparison between the results obtained from the 6-minute treatment and the 12-minute treatment, always based on the parameter a*.

**Table 15.1**

| **6-minute test** | | |
|---|---|---|
| | **T0** | **15 washes** |
| **a*** | 35.94 | 32.90 |

**Table 15.2**

| **12-minute test** | | |
|---|---|---|
| | **T0** | **15 washes** |
| **a*** | 33.62 | 31.18 |

Then it is verified the significance of variation between 6-minute treated locks and 12-minute treated locks:
Time 0 washes - 6-minute test vs 12-minute test: p = 0.02 -
Time 15 washes - 6-minute test vs 12-minute test: p = 0.03 -

In this case the difference is significant, but in favour of the 6-minute treatment, which shows better results in terms of parameter a* than the 12-minute treatment.

### 4.6. Comments

As regards the treatment of locks coloured with red dye, it can be observed a significant increase in the parameter a* (red intensity index), both for the locks subjected to the 6-minute treatment and for those subjected to the 12-minute treatment in comparison with the control locks.

The loss of colour is however evident both for the control locks and for the treated locks, if the values of a* are compared to time 0 and after 15 washes; however, this phenomenon is attenuated for the test locks, especially for the 6-minute treated locks, as shown by the photos.

### 5. Results - Gloss Evaluation

The assessment about the gloss of the locks refers to the concept of "saturation" or "chroma". It is calculated as [(a*)² + (b*)²]^{1/2}. The higher the saturation value, the more vivid and intense the colour.

The values obtained for the control locks are compared with those obtained for the treated locks.

### 5.1. 6-minute treatment - Brown

**Table 4: Saturation values of the control locks**

| **Saturation - Control** | | | | | | | | | |
|---|---|---|---|---|---|---|---|---|---|
| **Measurement** | **1** | **2** | **3** | **4** | **5** | **6** | **7** | **8** | **9** |
| **[(a*)²+(b*)²]^{1/2}** | 1.81 | 1.14 | 1.52 | 1.02 | 1.07 | 1.40 | 2.17 | 1.56 | 1.55 |

**Table 5: Saturation values of the treated/test locks**

| **Saturation** - **6-minute test** | | | | | | | | | |
|---|---|---|---|---|---|---|---|---|---|
| **Measurement** | **1** | **2** | **3** | **4** | **5** | **6** | **7** | **8** | **9** |
| **[(a*)²+(b*)²]^{1/2}** | 3.14 | 2.42 | 2.86 | 2.11 | 2.24 | 2.57 | 3.22 | 3.13 | 2.80 |

The average of the calculated values is as follows.

**Table 6**

| | **Control** | **6-minute test** |
|---|---|---|
| **Average saturation** | 1.47 | 2.72 |

Furthermore, it is evaluated whether the variation of saturation between treated locks and control locks is statistically significant by applying the t-test function. With values of p ≤ 0.05, the difference between the values is considered significant.

### 6-minute test vs control: p = 1.07 x 10⁻⁸

Therefore, it can be stated that the locks treated with oxygen for 6 minutes are significantly glossier than the control locks, in a highly significant way.

### 5.2. 6-minute treatment - Red

Below, the calculated saturation values for the control locks and for the locks treated for 6 minutes with the VL-1 device.

**Table 8: Saturation values of the control locks**

| **Saturation** - **Control** | | | | | | | | | |
|---|---|---|---|---|---|---|---|---|---|
| **Measurement** | **1** | **2** | **3** | **4** | **5** | **6** | **7** | **8** | **9** |
| **[(a*)²+(b*)²]^{1/2}** | 33.98 | 32.41 | 33.14 | 33.64 | 32.09 | 30.68 | 35.09 | 34.96 | 33.11 |

**Table 9: Saturation values of the treated/test locks**

| **Saturation - 6-minute test** | | | | | | | | | |
|---|---|---|---|---|---|---|---|---|---|
| **Measurement** | **1** | **2** | **3** | **4** | **5** | **6** | **7** | **8** | **9** |
| **[(a*)²+b*)²]^{1/2}** | 40.61 | 39.34 | 39.21 | 43.33 | 40.93 | 43.80 | 41.30 | 40.73 | 40.10 |

The average of the calculated values is as follows.

**Table 10**

| | **Control** | **6-minute test** |
|---|---|---|
| **Average saturation** | 33.23 | 41.04 |

Moreover, it is evaluated whether the variation of saturation between treated locks and control locks is significant:
6-minute test vs control: p = 9.6 x 10⁻⁶

Therefore, it can be stated that the locks treated with oxygen for 6 minutes are significantly glossier than the control locks, in a statistically very significant way.

### 5.3. Conclusions

Hair treated for 6-minutes with the VL-1 device in the post-dye phase is significantly glossier than the control locks, in a statistically very significant way. This result is equally noticeable both for locks dyed brown and for those dyed red.

The VL-1 device adopted is therefore able to significantly improve the visible gloss performance for hair dyes made with permanent dyes of different nature.

## Claims

1. A hair cosmetic method, comprising the steps of:
a) applying a hair care and beauty treatment to the hair;
b) supplying gaseous oxygen (O₂) to the hair, simultaneously and/or subsequently to the hair care and beauty treatment,
said hair care and beauty treatment being a colouring treatment,
wherein the gaseous oxygen has a concentration ≥ 80% (v/v) and the gaseous oxygen application phase has a duration comprised between 0.5 and 20 minutes.

2. Method according to claim 1, wherein the gaseous oxygen supply phase on the hair is simultaneous and subsequent to the colouring treatment.

3. Method according to claim 2, wherein the gaseous oxygen supply phase simultaneous to the colouring treatment occurs at the end of the time of pose of the colouring treatment.

4. Method according to claim 2 or 3, wherein the gaseous oxygen supply phase subsequent to the colouring treatment occurs after rinsing off the colouring treatment.

5. Method according to any one of claims 1 to 4, wherein the concentration of gaseous oxygen is comprised between 85% and 99% (v/v).

6. Method according to any one of claims 1 to 5, wherein the concentration of gaseous oxygen is comprised between 93% and 99% (v/v), preferably between 95% and 98% (v/v).

7. Method according to any one of claims 1 to 6, wherein the gaseous oxygen supply phase lasts between 2 and 20 minutes, preferably between 6 and 12-minutes.

8. Method according to any one of claims 1 to 7, wherein the gaseous oxygen supply is **characterized by** a flow ranging from 0.1 to 20 L/min.

9. Method according to claim 8, wherein the flow of gaseous oxygen is supplied at a pressure ranging from 0.1 to 3 bar.
